# FASCICULE DE BREVET EUROPEEN

(11) **EP 3 445 287 B1**
(45) Date de publication et mention de la délivrance du brevet: **01.04.2020**
(21) Numéro de dépôt: 17717455.4
(22) Date de dépôt: 19.04.2017
(51) Int. Cl.: A61F 2/16

(54) **DISPOSITIF D'INJECTION DE LENTILLE INTRAOCULAIRE SOUPLE ET NAVETTE DE STOCKAGE POUR SA MISE EN OEUVRE**
VORRICHTUNG ZUM INJIZIEREN EINER FLEXIBLEN INTRAOKULARLINSE UND SPEICHERSCHIFFCHEN ZUR DURCHFÜHRUNG DES VERFAHRENS
DEVICE FOR INJECTING A FLEXIBLE INTRAOCULAR LENS AND STORAGE SHUTTLE FOR IMPLEMENTING SAME

(30) Priorité: 21.04.2016 BE 201605271
(43) Date de publication de la demande: 27.02.2019
(73) Titulaire: PhysIOL SA, 4031 Liège (BE)
(72) Inventeur: PAGNOULLE, Christophe, 4800 Verviers (BE); BOZUKOVA, Dimitriya, 4031 Liège (BE); DELAY, Jean-Pascal Yves, 69130 Ecully (FR); VIDAL, Julien Laurent, 69006 Lyon (FR)
(74) Mandataire: Gevers Patents
(86) Numéro de dépôt international: PCT/EP2017/059265
(87) Numéro de publication internationale: WO 2017/182508

(56) Documents cités:
- US-A1- 2003 036 765
- US-A1- 2007 150 055
- US-A1- 2011 190 777
- US-A1- 2015 342 730

## Description

La présente invention est relative à un dispositif d'injection de lentille intraoculaire souple, comprenant
- un corps tubulaire présentant une enveloppe et une cavité axiale ainsi qu'une extrémité proximale et une extrémité distale,
- un poussoir introduit dans ladite cavité axiale, à l'extrémité proximale susdite, et déplaçable dans cette cavité suivant une direction axiale,
- un canon agencé à ladite extrémité distale du corps tubulaire et présentant une cavité d'éjection en communication avec la cavité axiale du corps tubulaire, cette cavité d'éjection ayant une section transversale décroissante en sens opposé au corps tubulaire, et
- un logement dans la cavité axiale du corps tubulaire destiné à recevoir une navette de stockage contenant une lentille intraoculaire souple.

Les lentilles intraoculaires (LIO) sont destinées au remplacement du cristallin (lentille naturelle) opacifié chez les patients atteints de cataracte, lors d'une intervention chirurgicale. Les interventions de la cataracte sont probablement les opérations les plus pratiquées chez les êtres humains, avec une amélioration significative de la vue chez 98 % des patients.

La technique de la chirurgie de la cataracte a accompli, ces dernières années, d'énormes progrès, grâce notamment à la diminution de la taille de l'incision par laquelle le cristallin est extrait et la lentille synthétique implantée dans l'œil. Les nouveaux matériaux souples (silicones, acryliques souples et hydrogels) permettent l'implantation de la lentille par une incision inférieure à celle pratiquée avec les premiers implants rigides en polyméthacrylate de méthyle (PMMA). La lentille souple peut en effet être implantée sous une conformation pliée ou enroulée, avant de recouvrer, une fois mise en place dans l'œil et grâce à ses propriétés viscoélastiques particulières (mémoire de forme), sa forme initiale et définitive. Les petites incisions, sans points de suture, présentent l'avantage de ne pas entraîner de déformation de la cornée et donc de prévenir l'astigmatisme. La récupération de la vue se fait plus rapidement et le résultat réfractif est plus stable.

Avec l'avènement des lentilles souples s'est posé le problème de la pose de ces implants. En effet, pour tirer parti d'une incision réduite, il faut pouvoir plier la lentille et l'introduire sous une forme enroulée dans l'œil. Une première technique connue consiste à implanter la lentille à l'aide d'une pince manuelle. La manipulation de l'implant est cependant fastidieuse. De surcroît, l'incision a dans ce cas une longueur minimale limite (de l'ordre de 3,2 mm) fixée par l'épaisseur des mors de la pince et celle de l'implant plié.

Une technique d'implantation alternative, de plus en plus répandue, consiste à utiliser un dispositif d'injection adapté aux lentilles souples. Ce dispositif d'injection est généralement constitué de deux éléments distincts, une cartouche, d'une part, et un injecteur, d'autre part (voir par exemple la demande de brevet US 2005/0065534). La cartouche permet le pliage, l'enroulement et le déplacement de la lentille enroulée vers un orifice de sortie d'un diamètre réduit au travers duquel l'implant est expulsé dans l'œil, l'injection de la lentille au travers de la cartouche étant assurée par un déplacement du poussoir de l'injecteur à la manière d'une seringue. Pour faciliter le déplacement de l'implant dans la cartouche, on fait parfois usage d'un poussoir muni à son extrémité d'un tampon cylindrique qui peut être introduit dans un conduit de la cartouche et quelques gouttes d'un additif lubrifiant sont généralement appliquées sur les faces internes de la cartouche. Il s'agit généralement d'un produit visqueux de la famille des polysaccharides (hyaluronate de sodium, cellulose méthylique d'hydroxypropyle, sulfate de chondroïtine...) dont l'usage est très répandu en chirurgie ophtalmologique. La technique de l'injection permet l'introduction de l'implant dans des incisions de dimensions pouvant être inférieures à 2 mm, tout en contribuant à une simplification et à une sécurisation de la procédure d'implantation.

De nombreuses variantes de telles cartouches sont actuellement disponibles et elles possèdent un orifice adapté à différentes tailles d'incision de forme circulaire ou polygonale. La principale limite inhérente à ces cartouches est liée à l'opération manuelle de positionnement de la lentille (chargement) dans la chambre de chargement. La lentille à implanter doit être retirée de son conditionnement et placée correctement dans la cartouche. La lentille est d'abord positionnée à plat au sein de la chambre de chargement à l'aide d'une pince et en exerçant une contrainte verticale sur l'implant. La cartouche est alors refermée manuellement, en ovalisant l'implant (pré-enroulement). Lors de cette opération de chargement de la lentille, le risque de pincer et d'abîmer l'implant lors de la fermeture est très élevé.

Très récemment, des dispositifs d'injection de lentilles intraoculaires, préchargés avec une lentille souple, ont été mis au point (voir par exemple EP-A-1481652). Ces systèmes d'injection intégrés, cartouche et injecteur ne formant qu'un seul et unique dispositif, servent également de conditionnement à la lentille et éliminent l'opération de chargement manuel de l'implant dans la cartouche et toutes les contraintes liées à cette manipulation. Le recours à un dispositif préchargé présente un gain de temps considérable lors de l'acte chirurgical et contribue à une implantation plus sécuritaire. Les sources de contamination de la lentille sont fortement réduites, les contacts entre les doigts du chirurgien et l'implant étant éliminés. Si ces dispositifs constituent une avancée technologique indéniable, il n'en reste pas moins qu'ils présentent tous de nombreuses limites.

Une des difficultés majeures est de permettre à la fois le stockage de la lentille dans une position non contrainte (à plat) et son enroulement au moment de l'implantation sous l'action du poussoir, et ce sans endommager l'implant. La lentille a en effet tendance à s'enrouler autour de l'extrémité du poussoir lors de l'injection, ce qui peut entraver l'expulsion de l'implant dans l'oeil et endommager la lentille.

Une solution consiste à utiliser un mécanisme de prépliage de la lentille très sophistiqué et intégré à l'injecteur. De cette manière, la lentille peut être mécaniquement prépliée, avant d'actionner le poussoir, ce qui évite un pincement de la lentille par le poussoir (voir par exemple FR-A-2808993). Les mécanismes de prépliage envisagés sont cependant peu performants et sources de défaillance.

Une autre limite de ces systèmes préchargés et intégrés (injecteur et cartouche), étroitement liée aux contraintes technologiques évoquées ci-dessus, réside dans la taille d'incision, généralement supérieure à 3,0 mm.

Un autre problème potentiel de conception est à prendre en considération dans le cas spécifique des lentilles intraoculaires hydrophiles (hydrogels). Ces dernières sont stérilisées en phase vapeur et doivent impérativement être stockées dans un milieu aqueux pour garantir le taux d'hydratation et la souplesse mécanique du matériau. Le système d'injection préchargé intégré est donc stérilisé en phase vapeur et livré immergé dans une solution aqueuse, ce qui rend très hasardeuse la manipulation du dispositif au moment de l'implantation (surface mouillée et préhension difficile) et peut également poser des problèmes de sélection des matériaux impliqués dans la conception de l'injecteur (compatibilité avec la stérilisation vapeur).

On connaît enfin des dispositifs d'injection de lentilles intraoculaires comprenant les deux éléments distincts, cartouche et injecteur, la cartouche étant préchargée de la lentille intraoculaire à injecter dans un récipient rempli d'un liquide stérile. De manière plus précise, la cartouche comprend typiquement deux parties soudées ou d'une seule pièce : un canon (ou embout) pour l'expulsion de la lentille et une chambre de stockage (ou navette de stockage). Lors de sa conservation, la lentille intraoculaire se trouve dans la chambre de stockage (ou chambre de chargement) de la cartouche et n'est pas à l'état contraint : elle conserve sa souplesse grâce au liquide du récipient. Dans ces dispositifs, on prélève la cartouche de son récipient à l'aide directement ou indirectement du dispositif d'injection lui-même, et après prélèvement, sous la poussée du poussoir du dispositif, la lentille s'enroule sur elle-même avant d'être éjectée dans l'œil d'un patient (voir par exemple BE-1016692, WO 2006/070219 et WO 2013/038021). Dans le BE-1016692, l'embout d'éjection (ou canon) du dispositif est conservé dans le récipient stérile, ce qui s'est avéré peu souhaitable, car le liquide du récipient peut être agressif vis-à-vis du matériau utilisé dans cet embout (ou canon) et en particulier pour un matériau de recouvrement interne du canon qui permet un glissement plus facile de la lentille le long du canon. Les demandes de brevet WO 2006/070219 et WO 2013/038021 divulguent des dispositifs extrêmement complexes, de fabrication coûteuse et nécessitant la mise en œuvre de mécanismes susceptibles de présenter des faiblesses lors de la manipulation. Tous deux nécessitent la prise en main de l'embout d'éjection (ou canon) qui est une partie particulièrement fragile du dispositif et dont il faut éviter à tout prix une contamination extérieure. Un système de chargement d'une lentille est aussi divulgué dans la demande de brevet US 2011/0190777.

### Résumé de l'invention

La présente comme définie dans les revendications 1, 8, 9, et 15 a pour but de surmonter les inconvénients précités et de mettre au point un dispositif d'injection de lentille intraoculaire simple dans lequel une navette de stockage contenant la lentille préchargée dans une position non contrainte puisse être prélevée d'un récipient stérile et chargée dans le corps du dispositif d'une manière simple, rapide et sans risque de contamination ou d'endommagement de l'embout d'injection (ou canon) du dispositif. Dans celui-ci, à l'état chargé, la lentille doit pouvoir être expulsée de la navette de stockage à l'état enroulé, sans risque d'endommagement.

On a résolu ces problèmes suivant l'invention par un dispositif d'injection tel que décrit au préambule, dans lequel l'enveloppe du corps tubulaire présente une ouverture latérale et dans lequel l'enveloppe susdite porte extérieurement en saillie un premier moyen de fixation rotative.

De préférence, ladite ouverture latérale donne accès de l'extérieur audit logement, de manière à permettre une introduction de la navette de stockage dans celui-ci.

De préférence, le premier moyen de fixation rotative est capable de coopérer avec un deuxième moyen de fixation rotative prévu sur la navette de stockage, de façon à permettre, en position de coopération des deux moyens de fixation rotative susdits, une rotation de la navette de stockage autour d'un axe de rotation parallèle à ladite direction axiale de la cavité axiale du corps tubulaire, entre une première position de la navette de stockage où elle est à l'extérieur du corps tubulaire et une deuxième position de la navette de stockage où elle est chargée dans le logement susdit.

Dans ce dispositif, il suffit d'une ouverture latérale dans le corps tubulaire pour donner accès au logement de réception de la navette de stockage. Les moyens de fixation rotative permettent une rotation sans problème de la navette de stockage depuis l'extérieur jusque dans le logement de réception autour d'un axe parallèle à la direction axiale du déplacement du poussoir, c'est-à-dire parallèle à l'axe du corps tubulaire. L'angle de rotation peut présenter de préférence une valeur comprise entre 90° et 180°. Dans ce logement la navette de stockage est prête pour l'expulsion de la lentille dans l'embout (ou canon) sous la poussée du poussoir actionnable par un opérateur. Dans une telle forme de réalisation le corps tubulaire, l'embout, l'ouverture latérale et le premier moyen de fixation rotative peuvent être réalisés en matière synthétique, notamment par moulage, sans nécessiter, pour la fabrication du dispositif en soi, un assemblage de nombreuses pièces par des moyens complexes tels que verrous, boutons de pivotement, languettes d'encliquetage, etc. L'embout (ou canon) est déjà en place avant l'introduction de la navette de stockage et ne doit donc plus être manipulé après l'introduction de la navette de stockage dans le corps tubulaire. Enfin l'embout peut être conservé avec le dispositif d'injection, au sec, et non dans un milieux aqueux stérile qui risque de l'endommager.

Suivant une forme de réalisation de l'invention, l'enveloppe du corps tubulaire porte, comme premier moyen de fixation rotative, un cylindre creux qui présente un axe longitudinal parallèle à ladite direction axiale, et dans lequel peut être enfoncée une tige prévue sur la navette de stockage, comme deuxième moyen de fixation rotative. Suivant une autre forme de réalisation de l'invention l'enveloppe du corps tubulaire porte, comme premier moyen de fixation rotative, une tige axialement parallèle à ladite direction axiale, et autour de laquelle peut être enfoncé un cylindre creux prévu sur la navette de stockage, comme deuxième moyen de fixation rotative. Dans ces formes de réalisation les deux moyens de fixation rotative forment ensemble les éléments d'un gond ou charnière qui tous deux sont disposés en saillie sur leur support respectifs et donc permettent une fabrication particulièrement aisée et une coopération par simple enfoncement d'un moyen de fixation dans l'autre.

Suivant une forme particulière de réalisation de l'invention le logement dans la cavité axiale du corps tubulaire présente des premiers moyens d'encliquetage capables de coopérer avec des deuxièmes moyens d'encliquetage prévus sur la navette de stockage de manière à bloquer la navette de stockage dans sa deuxième position susdite. Une fois introduite dans le logement de réception, la navette de stockage y est ainsi bloquée et ne peut plus se déplacer hors de la position où elle est prête pour l'expulsion de la lentille dans l'embout sous la poussée du poussoir.

De préférence, l'enveloppe porte extérieurement en saillie le premier moyen de fixation rotative à proximité de l'ouverture latérale. De préférence, cela signifie que le premier moyen de fixation rotative est située au même endroit le long de la direction axiale que l'ouverture latérale et le logement. Cela ressortira plus clairement de l'enseignement des dessins.

La présente invention concerne également une navette de stockage à insérer dans un dispositif d'injection de lentille intraoculaire souple suivant l'invention. Cette navette de stockage comprend
- un corps de navette présentant une cavité de navette destinée à recevoir une lentille intraoculaire souple,
- une ouverture de sortie, qui est destinée, dans la deuxième position susdite de la navette de stockage, à permettre une communication entre la cavité de navette et la cavité d'injection de l'embout, et
- une ouverture d'entrée, qui est destinée, dans la deuxième position susdite de la navette de stockage, à permettre une communication entre la cavité de navette et la cavité axiale du corps tubulaire dans laquelle se trouve ledit poussoir, et
le corps de navette porte extérieurement en saillie ledit deuxième moyen de fixation rotative capable de coopérer avec ledit premier élément de fixation rotative porté par l'enveloppe du corps tubulaire.

De préférence, le corps de navette porte extérieurement lesdits deuxièmes moyens d'encliquetage capables de coopérer avec lesdits premiers moyens d'encliquetage du logement du corps tubulaire du dispositif d'injection selon une variante préférée décrite, pour bloquer la navette de stockage dans sa deuxième position.

De préférence, la navette de stockage présente un moyen de préhension à saisir pour actionner ladite rotation de la navette de stockage entre sa première position et sa deuxième position.

De préférence la navette de stockage suivant l'invention est conservée dans un récipient stérile. Les inventeurs proposent également un tel récipient stérile comprenant une navette de stockage telle que décrite ci-dessus.

Avantageusement, pendant la conservation de la navette de stockage, ledit récipient stérile est pourvu d'une ouverture obturable. Il est de préférence rempli d'un milieu aqueux stérile.

Suivant une forme particulière de réalisation le récipient stérile présente une partie de retenue de navette de stockage dans laquelle le corps de navette est maintenu avec l'ouverture d'entrée de la navette de stockage et ledit deuxième moyen de fixation rotative de celle-ci, orientés vers l'ouverture obturable du récipient, et une partie d'enfoncement destinée à recevoir le corps tubulaire du dispositif d'injection, muni de son canon, de façon que, en position d'enfoncement dudit corps tubulaire dans cette partie d'enfoncement, ledit premier moyen de fixation rotative de celui-ci coopère avec ledit deuxième moyen de fixation rotative de la navette de stockage, en permettant ainsi un prélèvement de la navette de stockage hors du récipient. Dans ce récipient, le dispositif d'injection de lentille intraoculaire suivant l'invention peut être enfoncé avec son canon agencé à l'extrémité distale du corps tubulaire, sans risque de l'endommager. En outre la navette de stockage peut être prélevée du récipient et amenée dans le logement de réception de navette de stockage, sans manipulation de l'embout (ou canon).

Suivant une forme avantageuse de l'invention, la partie de retenue de la navette de stockage dans le récipient présente un fond nervuré permettant un passage du liquide susdit aussi bien par l'ouverture de sortie de la navette de stockage que par l'ouverture d'entrée de celle-ci. De cette manière la lentille intraoculaire en matériau souple est bien immergée dans le milieu aqueux stérile.

La présente invention concerne également un procédé de chargement d'une navette de stockage suivant l'invention dans un dispositif d'injection de lentille intraoculaire suivant l'invention. Ce procédé comprend les étapes suivantes :
- une ouverture du récipient stérile contenant la navette de stockage pourvue d'une lentille intraoculaire souple,
- une introduction du corps tubulaire du dispositif d'injection, muni de son canon, dans ladite partie d'enfoncement du récipient stérile, avec coopération entre le premier moyen de fixation rotative du corps tubulaire et le deuxième moyen de fixation rotative de la navette de stockage,
- un prélèvement hors du récipient du corps tubulaire et de la navette de stockage fixés l'un à l'autre,
- une rotation de la navette de stockage entre sa première position susdite à l'extérieur du corps tubulaire et sa deuxième position susdite où elle est chargée à l'intérieur du logement susdit du corps tubulaire par l'ouverture latérale susdite prévue dans le corps tubulaire, la lentille intraoculaire étant dans cette deuxième position de la navette de stockage prête à être déplacée et enroulée sous la poussée du poussoir dans la cavité d'éjection de l'embout, puis éjectée à l'extérieur.

D'autres détails et particularités de l'invention sont à prélever des revendications annexées.

### Brève description des figures

La présente invention va à présent être décrite de manière plus détaillée en faisant référence à des dessins, qui illustrent de manière non limitative, une forme de réalisation suivant l'invention.
La figure 1 est une vue en perspective d'un dispositif d'injection suivant l'invention, sans navette de stockage.
La figure 2 est une vue en perspective d'un récipient contenant une navette de stockage, après détachement de l'opercule fermant ce récipient.
La figure 3 représente une vue du dessus du récipient selon la figure 2.

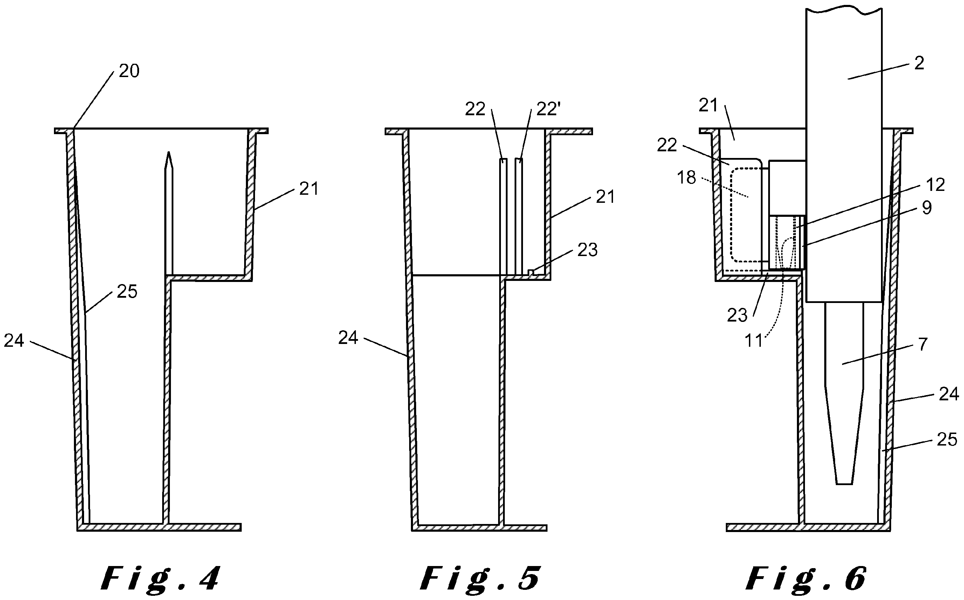

La figure 4 représente une vue en coupe suivant la ligne IV-IV de la figure 3.
La figure 5 représente une vue en coupe suivant la ligne V-V de la figure 3.
La figure 6 représente une vue en coupe suivant la ligne VI-VI de la figure 3 où la navette de stockage est en position de coopération avec le corps tubulaire du dispositif d'injection enfoncé dans le récipient stérile.
Les figures 7 et 8 sont des vues en perspective du dispositif d'injection selon la figure 1, avec la navette de stockage respectivement dans sa première position et dans sa deuxième position.

Sur les différents dessins, les éléments identiques ou analogues sont désignés par les mêmes références.

### Description détaillée de certains modes de réalisation de l'invention

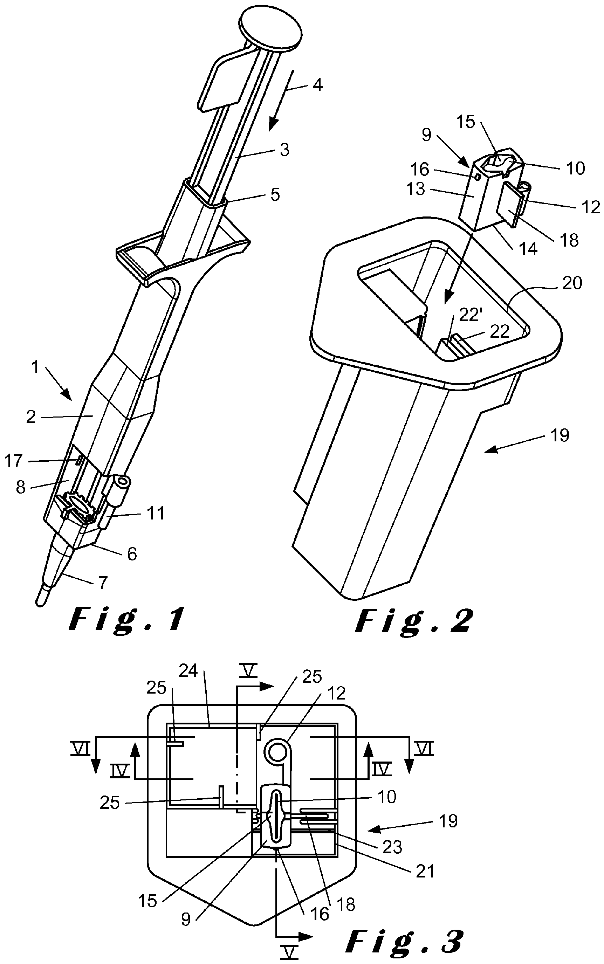

Comme on peut le voir sur la figure 1 le dispositif d'injection représenté comporte un corps tubulaire 1 présentant une enveloppe 2 renfermant une cavité axiale dans laquelle peut coulisser un poussoir 3 suivant une direction axiale 4. Le poussoir 3 est introduit dans la cavité axiale à l'extrémité proximale 5 du corps tubulaire 1. A l'extrémité distale 6 de celui-ci est agencé un canon 7 qui présente une cavité d'éjection en communication avec la cavité axiale du corps tubulaire 1. Cette cavité d'éjection présente une section transversale décroissante en sens opposé au corps tubulaire et est ouverte vers l'extérieur à l'extrémité libre du canon 7.

Le dispositif d'injection illustré comporte aussi un logement 8 dans la cavité axiale du corps tubulaire 1 qui est destiné à recevoir une navette de stockage 9 contenant une lentille intraoculaire souple 10 (voir figure 2).

Suivant l'invention l'enveloppe 2 du corps tubulaire 1 présente, comme illustré sur la figure 1, une ouverture latérale donnant accès au logement 8 à partir de l'extérieur. Par cette ouverture la navette de stockage 9 peut être introduite dans le dispositif. A proximité de cette ouverture, plus particulièrement au bord de celle-ci, l'enveloppe 2 porte en saillie, extérieurement, un premier moyen de fixation rotative, illustré ici sous la forme d'une tige cylindrique 11, qui présente un axe longitudinal parallèle à la direction axiale 4 du poussoir 3.

Cette tige cylindrique 11 est agencée de manière à pouvoir coopérer avec un deuxième moyen de fixation rotative, prévu en saillie, extérieurement, sur la navette de stockage 9. Dans la forme de réalisation illustrée, ce deuxième moyen de fixation rotative est un cylindre creux 12 capable de coopérer avec la tige cylindrique 11 par enfoncement, tout en permettant une rotation de la navette de stockage par rapport au dispositif d'injection. Enfoncés l'un dans l'autre, la tige 11 et le cylindre creux 12 forment une sortie du gond usuelle.

La navette de stockage 9 présente un corps de navette 13 qui porte extérieurement en saillie ce cylindre creux 12 et qui est pourvu d'une cavité destinée à recevoir la lentille intraoculaire souple 10 dans un état non contraint. La navette de stockage 9 présente une ouverture de sortie 14 destinée à permettre une communication entre la cavité de la navette de stockage 9 et la cavité d'éjection du canon 7, lorsque la navette de stockage 9 est chargée dans le logement 8 du dispositif d'injection. La navette de stockage 9 présente aussi une ouverture d'entrée 15 qui, dans cette position de la navette de stockage 9, permet une communication avec la cavité axiale du corps tubulaire 1 du dispositif.

Dans l'exemple illustré, le corps de navette 13 est pourvu d'un tenon 16 faisant saillie vers l'extérieur. Ce tenon 16 est disposé de façon que, dans la position de chargement de la navette de stockage dans le logement 8, il s'encliquète dans un évidement 17 de l'enveloppe 2 de façon à bloquer la navette de stockage dans ce logement. D'autres moyens d'encliquetage usuels pour l'homme de métier peuvent évidemment être conçus au lieu de ce tenon 16 et de cet évidement 17.

Le corps de navette 13 porte aussi sur une de ses faces une languette 18 qui sert de moyen de préhension pour saisir la navette de stockage 9 et la faire pivoter autour de la tige 11 du corps tubulaire entre une première position, située à l'extérieur du corps tubulaire 1, et une deuxième position située à l'intérieur du logement 8.

Comme représenté sur les figures 2 et 3, la navette de stockage 9 est conservée dans un récipient stérile 19, qui présente une ouverture 20 obturable par exemple par un opercule détachable non représenté. A l'état obturé, le récipient stérile contient un milieu aqueux stérile approprié pour que la lentille intraoculaire 10 conserve sa souplesse.

Le récipient 19 de l'exemple illustré comporte une partie de retenue de navette de stockage 21 dans laquelle le corps de navette est maintenu avec l'ouverture d'entrée 15 et le cylindre creux 12 orientés vers le haut, c'est-à-dire vers l'ouverture obturable de récipient. Pour guider l'introduction de la navette de stockage dans cette partie de retenue 21, le récipient comporte des guidages 22, 22' entre lesquels peut coulisser la languette de préhension 18. Le fond de cette partie de retenue peut être pourvu d'une ou de plusieurs nervures 23. Cet agencement favorise la circulation du milieu aqueux stérile dans la navette de stockage 9 aussi bien par l'ouverture d'entrée 15 que par l'ouverture de sortie 14 de celle-ci, quand la navette de stockage 9 est conservée dans le récipent stérile 19.

Le récipient 19 de l'exemple illustré comporte en outre une partie d'enfoncement 24 pour le dispositif d'injection muni de son canon 7. En position d'enfoncement la tige 11 s'enfonce dans le cylindre creux 12 porté par la navette de stockage 9. Pour faciliter cet enfoncement, des nervures de guidage 25 conduisent les deux éléments de fixation rotative 11 et 12 à coopérer parfaitement. Le canon 7 (ou embout), déjà en place sur le dispositif d'injection, n'entre ainsi pas en contact avec les parois du récipient et il n'atteint pas le fond de la partie d'enfoncement. Son introduction dans le récipient se fait donc sans risque d'endommagement, ni de contamination.

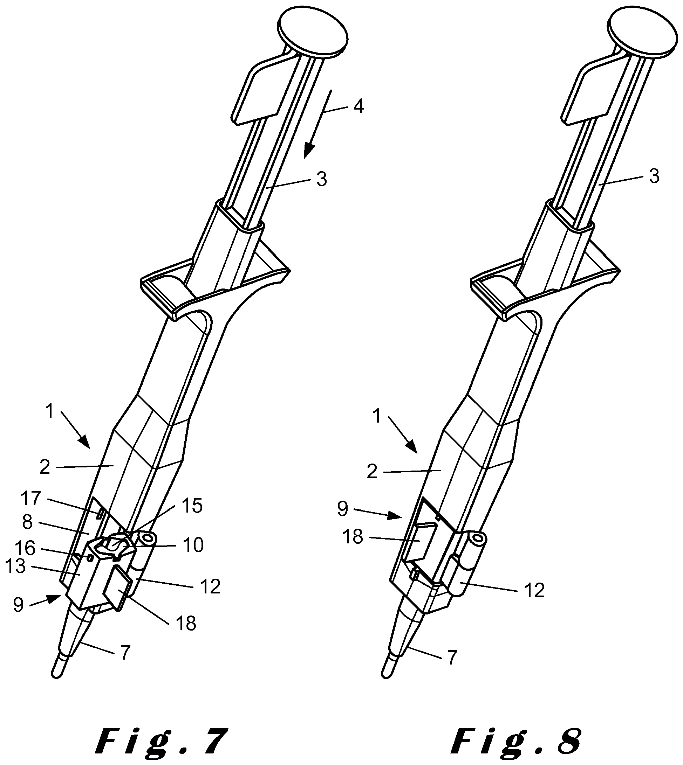

Lorsqu'on extrait le dispositif d'injection hors du récipient stérile 19, il emporte avec lui la navette de stockage 9 qui se trouve alors dans la position illustrée sur la figure 7.

L'opérateur saisit alors la languette de préhension 18 et fait pivoter la navette de stockage 9 dans le logement 8 où elle est bloquée par les moyens d'encliquetage 16 et 17.

Dans cette position d'encliquetage, l'ouverture d'entrée 15 de la navette de stockage communique avec la cavité axiale du corps tubulaire 1. L'opérateur peut alors effectuer une poussée sur le poussoir 3, qui à son extrémité distale est avantageusement muni d'un coussin en matière souple pour ne pas endommager la lentille. Sous l'action du poussoir la lentille 10 est alors poussée à travers l'ouverture de sortie 14 de la navette de stockage dans la cavité d'éjection du canon 7. Celle-ci, présentant une section transversale décroissante en direction de l'extrémité libre du canon 7, force la lentille à s'enrouler sur elle-même, ce qui permet de l'éjecter sous cette forme enroulée dans l'œil du patient.

Il doit être entendu que l'invention n'est en aucune façon limitée à cette forme de réalisation et que bien des modifications ou variantes peuvent y être apportées dans le cadre des revendications annexées.

## Revendications

1. Dispositif d'injection de lentille intraoculaire souple, comprenant
- un corps tubulaire (1) présentant une enveloppe (2) et une cavité axiale ainsi qu'une extrémité proximale (5) et une extrémité distale (6),
- un poussoir (3) introduit dans ladite cavité axiale, à l'extrémité proximale (5) susdite, et déplaçable dans cette cavité suivant une direction axiale (4),
- un canon (7) agencé à ladite extrémité distale (6) du corps tubulaire (1) et présentant une cavité d'éjection en communication avec la cavité axiale du corps tubulaire (1), cette cavité d'éjection ayant une section transversale décroissante en sens opposé au corps tubulaire (1), et
- un logement (8) dans la cavité axiale du corps tubulaire (1) destiné à recevoir une navette de stockage (9) contenant une lentille intraoculaire souple (10),
**caractérisé en ce que** l'enveloppe (2) du corps tubulaire présente une ouverture latérale, qui donne accès de l'extérieur audit logement (8), de manière à permettre une introduction de la navette de stockage (9) dans celui-ci, et **en ce que**, l'enveloppe (2) susdite porte extérieurement en saillie un premier moyen de fixation rotative (11) capable de coopérer avec un deuxième moyen de fixation rotative (12) prévu sur la navette de stockage (9), de façon à permettre, en position de coopération des deux moyens de fixation rotative susdits, une rotation de la navette de stockage (9) autour d'un axe de rotation parallèle à ladite direction axiale (4) de la cavité axiale du corps tubulaire (1), entre une première position de la navette de stockage (9) où elle est à l'extérieur du corps tubulaire (1) et une deuxième position de la navette de stockage (9) où elle est chargée dans le logement (8) susdit.

2. Dispositif d'injection suivant la revendication 1, dans lequel l'enveloppe (2) du corps tubulaire (1) porte, comme premier moyen de fixation rotative, un cylindre creux qui présente un axe longitudinal parallèle à ladite direction axiale (4), et dans lequel peut être enfoncée une tige prévue sur la navette de stockage (9), comme deuxième moyen de fixation rotative.

3. Dispositif d'injection suivant la revendication 1, dans lequel l'enveloppe (2) du corps tubulaire (1) porte, comme premier moyen de fixation rotative, une tige (11) axialement parallèle à ladite direction axiale (4), et autour de laquelle peut être enfoncé un cylindre creux (12) prévu sur la navette de stockage (9), comme deuxième moyen de fixation rotative.

4. Dispositif d'injection suivant l'une quelconque des revendications précédentes, **caractérisé en ce que** le logement (8) dans la cavité axiale du corps tubulaire (1) présente des premiers moyens d'encliquetage (17) capables de coopérer avec des deuxièmes moyens d'encliquetage (16) prévus sur la navette de stockage (9) de manière à bloquer la navette de stockage (9) dans sa deuxième position susdite.

5. Navette de stockage (9) pour dispositif d'injection intraoculaire souple suivant l'une quelconque des revendications 1 à 4, **caractérisée en ce qu'**elle comprend
- un corps de navette (13) présentant une cavité de navette destinée à recevoir une lentille intraoculaire souple (10),
- une ouverture de sortie (14), qui est destinée, dans la deuxième position susdite de la navette de stockage (9), à permettre une communication entre la cavité de navette et la cavité d'éjection du canon (7), et
- une ouverture d'entrée (15), qui est destinée, dans la deuxième position susdite de la navette de stockage (9), à permettre une communication entre la cavité de navette et la cavité axiale du corps tubulaire (1) dans laquelle se trouve ledit poussoir (3), et
**en ce que** le corps de navette (13) porte extérieurement en saillie ledit deuxième moyen de fixation rotative (12) capable de coopérer avec ledit premier élément de fixation rotative (11) porté par l'enveloppe (2) du corps tubulaire (1).

6. Navette de stockage (9) suivant la revendication 5, **caractérisée en ce que** le corps de navette (13) porte extérieurement lesdits deuxièmes moyens d'encliquetage (16) capables de coopérer avec lesdits premiers moyens d'encliquetage (17) du logement (8) du corps tubulaire (1) du dispositif d'injection selon la revendication 4, pour bloquer la navette de stockage (9) dans sa deuxième position susdite.

7. Navette de stockage (9) suivant l'une ou l'autre des revendications 5 et 6, **caractérisée en ce qu'**elle présente un moyen de préhension (18) à saisir pour actionner ladite rotation de la navette de stockage (9) entre sa première position et sa deuxième position.

8. Ensemble d'injection comprenant un dispositif d'injection selon l'une quelconque des revendications 1 à 4 et une navette de stockage (9) selon l'une quelconque des revendications 5 à 7.

9. Récipient stérile (19) comprenant une navette de stockage (9) selon l'une quelconque des revendications 5 à 7.

10. Récipient stérile (19) suivant la revendication précédente, **caractérisé en ce que** le récipient stérile (19) est pourvu d'une ouverture obturable (20).

11. Récipient stérile (19) suivant la revendication 10, **caractérisé en ce que** le récipient stérile (19) présente une partie de retenue de navette (21) dans laquelle le corps de navette (13) est maintenu avec l'ouverture d'entrée (15) de la navette de stockage (9) et ledit deuxième moyen de fixation rotative (12) de celle-ci, orientés vers l'ouverture obturable (20) du récipient (19), et une partie d'enfoncement (24) destinée à recevoir le corps tubulaire (1) du dispositif d'injection, muni de son canon (7), de façon que, en position d'enfoncement dudit corps tubulaire (1) dans cette partie d'enfoncement (24), ledit premier moyen de fixation rotative (11) de celui-ci coopère avec ledit deuxième moyen de fixation rotative (12) de la navette de stockage (9), en permettant ainsi un prélèvement de la navette de stockage (9) hors du récipient stérile (19).

12. Récipient stérile (19) suivant la revendication précédente, **caractérisé en ce qu'**il comprend en outre un milieu aqueux stérile.

13. Récipient stérile (19) suivant la revendication précédente, **caractérisé en ce que** la partie de retenue de navette (21) présente un fond nervuré permettant un passage de liquide du milieu aqueux aussi bien par l'ouverture de sortie (14) de la navette de stockage (9) que par l'ouverture d'entrée (15) de celle-ci.

14. Récipient stérile (19) suivant l'une des revendications 11 à 13, **caractérisé en ce que**, dans ladite position d'enfoncement du corps tubulaire (1) dans le récipient stérile (19), le canon (7) est sans contact avec le récipient (19).

15. Procédé de chargement d'une navette de stockage (9) suivant l'une quelconque des revendications 5 à 7 dans un dispositif d'injection de lentille intraoculaire souple suivant l'une quelconque des revendications 1 à 4, **caractérisé en ce qu'**il comprend
- une ouverture d'un récipient stérile (19) contenant la navette de stockage (9) pourvue d'une lentille intraoculaire souple,
- une introduction du corps tubulaire (1) du dispositif d'injection, muni de son canon (7), dans ladite partie d'enfoncement du récipient stérile (19), avec coopération entre le premier moyen de fixation rotative (11) du corps tubulaire (1) et le deuxième moyen de fixation rotative (12) de la navette de stockage (9),
- un prélèvement hors du récipient stérile (19) du corps tubulaire (1) et de la navette de stockage (9) fixés l'un à l'autre,
- une rotation de la navette de stockage (9) entre sa première position susdite à l'extérieur du corps tubulaire (1) et sa deuxième position susdite où elle est chargée à l'intérieur du logement (8) susdit du corps tubulaire (1) par l'ouverture latérale susdite prévue dans le corps tubulaire (1), la lentille intraoculaire étant dans cette deuxième position de la navette de stockage (9) prête à être déplacée et enroulée sous la poussée du poussoir (3) dans la cavité d'éjection du canon (7), puis apte à être éjectée à l'extérieur.

## Patentansprüche

1. Vorrichtung zum Injizieren einer flexiblen Intraokularlinse, umfassend
- einen rohrförmigen Körper (1) mit einem Gehäuse (2) und einem axialen Hohlraum sowie einem proximalen Ende (5) und einem distalen Ende (6),
- einen Schieber (3), der in den genannten axialen Hohlraum am vorgenannten proximalen Ende (5) eingesetzt und in diesem Hohlraum in axialer Richtung (4) verschiebbar ist,
- einen Tubus (7), der an dem genannten distalen Ende (6) des rohrförmigen Körpers (1) angeordnet ist und einen Ausstoßhohlraum aufweist, der mit dem axialen Hohlraum des rohrförmigen Körpers (1) in Verbindung steht, wobei dieser Ausstoßhohlraum einen Querschnitt aufweist, der in der zum rohrförmigen Körper (1) entgegengesetzten Richtung abnimmt,
und
- eine Aussparung (8) in dem axialen Hohlraum des röhrenförmigen Körpers (1) zur Aufnahme eines Speichershuttles (9), das eine flexible Intraokularlinse (10) enthält,
**dadurch gekennzeichnet, dass** das Gehäuse (2) des rohrförmigen Körpers eine seitliche Öffnung aufweist, die von außen Zugang zu der genannten Aussparung (8) gewährt, um ein Einführen des Speichershuttles (9) in diese Aussparung zu ermöglichen, und dass das vorgenannte Gehäuse (2) nach außen vorstehend ein erstes Drehbefestigungsmittel (11) trägt, das in der Lage ist, mit einem zweiten Drehbefestigungsmittel (12), welches an dem Speichershuttle (9) vorgesehen ist, zusammenzuwirken, so dass in der Position des Zusammenwirkens der beiden vorgenannten Drehbefestigungsmittel eine Drehung des Speichershuttles (9) um eine Drehachse parallel zu der genannten axialen Richtung (4) des axialen Hohlraums des rohrförmigen Körpers (1) zwischen einer ersten Position des Speichershuttles (9), in der sich das Shuttle außerhalb des rohrförmigen Körpers (1) befindet, und einer zweiten Position des Speichershuttles (9), in der das Shuttle in der vorgenannten Aussparung (8) bestückt wird, ermöglicht wird.

2. Injektionsvorrichtung nach Anspruch 1, bei der das Gehäuse (2) des rohrförmigen Körpers (1) als erstes Drehbefestigungsmittel einen Hohlzylinder trägt, der eine zu der genannten axialen Richtung (4) parallele Längsachse aufweist, und in den ein am Speichershuttle (9) vorgesehener Stift als zweites Drehbefestigungsmittel eingetrieben werden kann.

3. Injektionsvorrichtung nach Anspruch 1, bei der das Gehäuse (2) des rohrförmigen Körpers (1) als erstes Drehbefestigungsmittel einen zu der genannten axialen Richtung (4) achsparallelen Stift (11) trägt, und um den ein am Speichershuttle (9) vorgesehener Hohlzylinder (12) als zweites Drehbefestigungsmittel eingetrieben werden kann.

4. Injektionsvorrichtung nach einem der vorhergehenden Ansprüche, **dadurch gekennzeichnet, dass** die Aussparung (8) in dem axialen Hohlraum des rohrförmigen Körpers (1) erste Einrastmittel (17) aufweist, die in der Lage sind, mit zweiten Einrastmitteln (16) zusammenzuwirken, die an dem Speichershuttle (9) vorgesehen sind, um das Speichershuttle (9) in seiner zweiten vorgenannten Position zu arretieren.

5. Speichershuttle (9) für eine flexible intraokulare Injektionsvorrichtung nach einem der Ansprüche 1 bis 4, **dadurch gekennzeichnet, dass** das Shuttle umfasst
- einen Shuttlekörper (13) mit einem Shuttlehohlraum zur Aufnahme einer flexiblen Intraokularlinse (10),
- eine Auslassöffnung (14), die dazu bestimmt ist, in der vorgenannten zweiten Position des Speichershuttles (9) eine Verbindung zwischen dem Hohlraum des Shuttles und dem Ausstoßhohlraum des Tubus (7) zu ermöglichen, und
- eine Einlassöffnung (15), die dazu bestimmt ist, in der vorgenannten zweiten Position des Speichershuttles (9) eine Verbindung zwischen dem Hohlraum des Shuttles und dem axialen Hohlraum des rohrförmigen Körpers (1), in dem sich der genannte Schieber (3) befindet, zu ermöglichen, und
dass der Shuttlekörper (13) nach außen vorstehend das genannte zweite Drehbefestigungsmittel (12) trägt, das in der Lage ist, mit dem genannten ersten Drehbefestigungsmittel (11), welches von dem Gehäuse (2) des rohrförmigen Körpers (1) getragen wird, zusammenzuwirken.

6. Speichershuttle (9) nach Anspruch 5, **dadurch gekennzeichnet, dass** der Shuttlekörper (13) außen die genannten zweiten Einrastmittel (16) trägt, die in der Lage sind, mit den genannten ersten Einrastmitteln (17) der Aussparung (8) des rohrförmigen Körpers (1) der Injektionsvorrichtung nach Anspruch 4 zusammenzuwirken, um das Speichershuttle (9) in seiner zweiten vorgenannten Position zu arretieren.

7. Speichershuttle (9) nach dem einen oder anderen der Ansprüche 5 und 6, **dadurch gekennzeichnet, dass** das Shuttle ein Griffstück (18) aufweist, das zum Auslösen der genannten Drehung des Speichershuttles (9) zwischen seiner ersten Position und seiner zweiten Position zu betätigen ist.

8. Injektionsbaugruppe mit einer Injektionsvorrichtung nach einem der Ansprüche 1 bis 4 und einem Speichershuttle (9) nach einem der Ansprüche 5 bis 7.

9. Steriler Behälter (19) mit einem Speichershuttle (9) nach einem der Ansprüche 5 bis 7.

10. Steriler Behälter (19) nach dem vorhergehenden Anspruch, **dadurch gekennzeichnet, dass** der sterile Behälter (19) mit einer verschließbaren Öffnung (20) versehen ist.

11. Steriler Behälter (19) nach Anspruch 10, **dadurch gekennzeichnet, dass** der sterile Behälter (19) einen Shuttle-Rückhaltebereich (21) aufweist, in dem der Shuttlekörper (13) so gehalten wird, dass die Einlassöffnung (15) des Speichershuttles (9) und dessen genanntes zweites Drehbefestigungsmittel (12) der verschließbaren Öffnung (20) des Behälters (19) zugewandt sind, und einen Einbringbereich (24) aufweist, der dazu bestimmt ist, den rohrförmigen Körper (1) der Injektionsvorrichtung mit seinem Tubus (7) aufzunehmen, so dass in der Einbringposition des genannten rohrförmigen Körpers (1) in diesem Einbringbereich (24) dessen genanntes erstes Drehbefestigungsmittel (11) mit dem genannten zweiten Drehbefestigungsmittel (12) des Speichershuttles (9) zusammenwirkt, wodurch eine Entnahme des Speichershuttles (9) aus dem sterilen Behälter (19) ermöglicht wird.

12. Steriler Behälter (19) nach dem vorhergehenden Anspruch, **dadurch gekennzeichnet, dass** der Behälter ferner ein steriles wässriges Medium umfasst.

13. Steriler Behälter (19) nach dem vorhergehenden Anspruch, **dadurch gekennzeichnet, dass** der Shuttle-Rückhaltebereich (21) einen gerillten Boden aufweist, der einen Flüssigkeitsdurchlass des wässrigen Mediums sowohl durch die Auslassöffnung (14) des Speichershuttles (9) als auch durch dessen Einlassöffnung (15) ermöglicht.

14. Steriler Behälter (19) nach einem der Ansprüche 11 bis 13, **dadurch gekennzeichnet, dass** in der genannten Einbringposition des rohrförmigen Körpers (1) in den sterilen Behälter (19), der Tubus (7) mit dem Behälter (19) nicht in Berührung kommt.

15. Verfahren zum Laden eines Speichershuttles (9) nach einem der Ansprüche 5 bis 7 in eine Vorrichtung zum Injizieren einer flexiblen Intraokularlinse nach einem der Ansprüche 1 bis 4, **dadurch gekennzeichnet, dass** das Verfahren umfasst
- eine Öffnung eines sterilen Behälters (19), der das mit einer flexiblen Intraokularlinse versehene Speichershuttle (9) enthält, eine Einführung des rohrförmigen Körpers (1) der Injektionsvorrichtung mit seinem Tubus (7) in den genannten Einbringbereich des sterilen Behälters (19), wobei das erste Drehbefestigungsmittel (11) des rohrförmigen Körpers (1) und das zweite Drehbefestigungsmittel (12) des Speichershuttles (9) zusammenwirken,
- eine Entnahme des rohrförmigen Körpers (1) und des an ihm befestigten Speichershuttles (9) aus dem sterilen Behälter (19),
- eine Drehung des Speichershuttles (9) zwischen seiner vorgenannten ersten Position außerhalb des rohrförmigen Körpers (1) und seiner vorgenannten zweiten Position, in der das Shuttle im Inneren der vorgenannten Aussparung (8) des rohrförmigen Körpers (1) durch die vorgenannte seitliche Öffnung, die im rohrförmigen Körper (1) vorgesehen ist, geladen wird, wobei die Intraokularlinse in dieser zweiten Position des Speichershuttles (9) bereit ist, unter dem Druck des Schiebers (3) in den Ausstoßhohlraum des Tubus (7) bewegt und eingerollt zu werden und dann nach außen ausgestoßen werden kann.

## Claims

1. Soft intraocular lens injection device, comprising
- a tubular body (1) presenting a casing (2) and an axial cavity, as well as a proximal end (5) and a distal end (6),
- a pushbutton (3) introduced in said axial cavity, at the abovesaid proximal end (5), and moveable in this cavity along an axial direction (4),
- a barrel (7) arranged at said distal end (6) of the tubular body (1) and presenting an ejection cavity in communication with the axial cavity of the tubular body (1), this ejection cavity having a transversal cross-section decreasing in the direction opposite the tubular body (1), and
- a housing (8) in the axial cavity of the tubular body (1) intended to receive a storage container (9) containing a soft intraocular lens (10),
**characterised in that** the casing (2) of the tubular body presents a side opening, which gives access to the outside of said housing (8), so as to make it possible to introduce the storage container (9) in it, and **in that**, the abovesaid casing (2) carries, protruding externally, a first rotating fixing means (11), capable of engaging with a second rotating fixing means (12) provided on the storage container (9), so as to make it possible, in the engaging position of the two abovesaid rotating fixing means, a rotation of the storage container (9) about an axis of rotation parallel to said axial direction (4) of the axial cavity of the tubular body (1), between a first position of the storage container (9) where it is outside of the tubular body (1) and a second position of the storage container (9) where it is loaded into the abovesaid housing (8).

2. Injection device according to claim 1, wherein the casing (2) of the tubular body (1) carries, as a first rotating fixing means, a hollow cylinder which presents a longitudinal axis parallel to said axial direction (4), and where a rod provided on the storage container (9) can be sunken, as a second rotating fixing means.

3. Injection device according to claim 1, wherein the casing (2) of the tubular body (1) carries, as a first rotating fixing means, a rod (11) axially parallel to said axial direction (4), and about which a hollow cylinder (12) provided on the storage container (9) can be sunken, as a second rotating fixing means.

4. Injection device according to any one of the preceding claims, **characterised in that** the housing (8) in the axial cavity of the tubular body (1) presents first snap-fitting means (17), capable of engaging with second snap-fitting means (16) provided on the storage container (9) so as to block the storage container (9) in its abovesaid second position.

5. Storage container (9) for soft intraocular lens injection device according to any one of claims 1 to 4, **characterised in that** it comprises
- a container body (13) presenting a container cavity intended to receive a soft intraocular lens (10),
- an outlet opening (14), which is intended, in the abovesaid second position of the storage container (9), to make it possible for a communication between the container cavity and the ejection cavity of the barrel (7), and
- an inlet opening (15), which is intended, in the abovesaid second position of the storage container (9), to make it possible for a communication between the container cavity and the axial cavity of the tubular body (1) wherein said pushbutton (3) is located, and
**in that** the container body (13) carries, protruding externally, said second rotating fixing means (12), capable of engaging with said first rotating fixing element (11) carried by the casing (2) of the tubular body (1).

6. Storage container (9) according to claim 5, **characterised in that** the container body (13) carries externally, said second snap-fitting means (16), capable of engaging with said first snap-fitting means (17) of the housing (8) of the tubular body (1) of the injection device according to claim 4, to block the storage container (9) in its abovesaid second position.

7. Storage container (9) according to either of claims 5 and 6, **characterised in that** it presents a gripping means (18) to grip to actuate said rotation of the storage container (9) between its first position and its second position.

8. Injection assembly comprising an injection device according to any one of claims 1 to 4 and a storage container (9) according to any one of claims 5 to 7.

9. Sterile vessel (19) comprising a storage container (9) according to any one of claims 5 to 7.

10. Sterile vessel (19) according to the preceding claim, **characterised in that** the sterile vessel (19) is provided with a blockable opening (20).

11. Sterile vessel (19) according to claim 10, **characterised in that** the sterile vessel (19) presents a portion for retaining the container (21), wherein the container body (13) is maintained with the inlet opening (15) of the storage container (9) and said second rotating fixing means (12) of it, oriented towards the blockable opening (20) of the vessel (19), and a sinking portion (24) intended to receive the tubular body (1) of the injection device, equipped with its barrel (7), such that, in the sinking position of said tubular body (1) in this sinking portion (24), said first rotating fixing means (11) of it engages with said second rotating fixing means (12) of the storage container (9), by thus making it possible for a removal of the storage container (9) outside of the sterile vessel (19).

12. Sterile vessel (19) according to the preceding claim, **characterised in that** it further comprises a sterile aqueous environment.

13. Sterile vessel (19) according to the preceding claim, **characterised in that** the retaining portion of the container (21) presents a grooved base making it possible for a passage of liquid from the aqueous environment, both through the outlet opening (14) of the storage container (9), and through the inlet opening (15) of it.

14. Sterile vessel (19) according to one of claims 11 to 13, **characterised in that**, in said sinking position of the tubular body (1) in the sterile vessel (19), the barrel (7) has no contact with the vessel (19).

15. Method for loading a storage container (9) according to any one of claims 5 to 7 in a soft intraocular lens injection device according to any one of claims 1 to 4, **characterised in that** it comprises
- an opening of a sterile vessel (19) containing the storage container (9) provided with a soft intraocular lens,
- an introduction of the tubular body (1) of the injection device, equipped with its barrel (7), in said sinking portion of the sterile vessel (19), with engagement between the first rotating fixing means (11) of the tubular body (1) and the second rotating fixing means (12) of the storage container (9),
- a removal outside of the sterile vessel (19) of the tubular body (1) and of the storage container (9) fixed to one another,
- a rotation of the storage container (9) between its abovesaid first position outside of the tubular body (1) and its abovesaid second position where it is loaded inside the abovesaid housing (8) of the tubular body (1) through the abovesaid side opening provided in the tubular body (1), the intraocular lens being in this second position of the storage container (9) ready to be moved and wound under the push of the pushbutton (3) in the ejection cavity of the barrel (7), then capable of being ejected outside.
